# EUROPEAN PATENT SPECIFICATION

(11) **EP 0 487 716 B1**
(45) Date of publication and mention of the grant of the patent: **27.09.1995**
(21) Application number: 91912823.1
(22) Date of filing: 25.06.1991
(51) Int. Cl.: A61F 11/08

(54) **HEARING PROTECTIVE EARPLUG**
OHRSTÖPSEL ALS GEHÖRSCHUTZ
BOUCHON PROTECTEUR AURICULAIRE

(30) Priority: 25.06.1990 US 542775
(43) Date of publication of application: 03.06.1992
(73) Proprietor: CABOT CSC CORPORATION, Southbridge, MA 01550 (US)
(72) Inventor: GARDNER, Ross, Jr., Indianapolis, IN 46268 (US)
(74) Representative: Attfield, Donald James
(86) International application number: US9104538
(87) International publication number: WO9200049

(56) References cited:
- CH-A- 120 642
- DE-A- 424 682
- DE-A- 3 304 362
- US-A- 29 487
- US-A- 4 314 553
- US-A- 4 338 929
- US-A- 4 774 938
- US-A- 4 867 149

## Description

### BACKGROUND OF THE INVENTION

The present invention relates generally to a hearing protective earplug as defined in the preamble of Claim 1. Such an earplug is disclosed in DE-A-3 304 362.

Premolded single-flange earplugs composed of resilient polymeric solid materials such as natural rubber or plasticized polyvinylchloride are well known in the art. Perhaps the most commercially prevalent embodiment of such an earplug is that developed during World War II and designated as the "V51R" earplug. The "V51R" earplug comprises a forwardmost flange element disposed rearwardly over and supported by a relatively bulky central stalk member. Further details concerning the construction of the "V51R" earplug can be had by reference to U.S. Patent No. 2,393,005, to P.S. Veneklasen, January 15, 1946. A major problem incurred with premolded earplugs in general and as is exemplified in the particular case of the "V51R" earplug resides in the anatomical fact that the human ear canal is quite variable in size and geometry and, as a result, a single size of premolded single-flange earplugs available in the prior art have been found incapable of accommodating the broad range of human ear canal sizes. Therefore, the "V51R" earplug is presently commercially manufactured and supplied in five sizes - very small (VS), small (S), medium (M), large (L) and very large (VL). Several hearing protection experts have expressed the view that an additional, even larger, sixth size is warranted. This need for manufacture in multiple sizes does, of course, increase the complexity and cost of manufacture of presently known single-flange earplugs. In addition, few members of the general public have the knowledge and skill required to properly fit themselves with the appropriate size of earplug. Thus, yet another desideratum in the selection of a premolded single-flange earplug is that they be fitted to the individual wearer by a skilled hearing protection professional, thereby adding further complexity, time and expense to the selection of an appropriate size of such an earplug for individual users.

A beneficial aspect of the "V51R" earplug with its single rearwardly oriented flange is that human ear canals are generally of ovoid cross section and, as the "V51R" flange comes into contact with the narrowest opposed walls of the ovoid ear canal upon insertion, it readily compresses in that direction and expands in the direction normal thereto, in other words, in the direction of the largest dimension of the canal. As a result, only a modicum of insertion force is usually required to achieve an initial acoustic seal of the ear canal in the case of the "V51R" earplug. However, that seal can be readily broken, such as by jaw or head movement, particularly if the particular plug selected is of a size which does not precisely fit the ear canal. Accordingly, cognoscenti in the use of single-flange premolded earplugs generally adhere to an understanding that, for optimum hearing protection and for reduction of the problem of loss of the acoustic seal, the size of the plug selected should be just large enough to cause some discomfort to the wearer.

Additionally, the extent of the available deflection afforded the rearwardly oriented flange of the "V51R" earplug is markedly circumscribed by: (a) the tendency of a solid polymer flange to pucker or fold upon itself when inserted into an excessively small or ovoid orifice, and (b) the relatively large and stiff underlying support stalk member therefor. Thus, even with proper selection of size, the "V51R" plug flange can be found incapable of properly and comfortably accommodating ear canals of severely ovoid cross section.

In at least partial response to the foregoing problems, premolded earplugs having multiple solid polymer flanges of serially increasing diameters spaced apart along a stalk support member have been designed and manufactured. Several of these earplugs have been found to provide better and more secure acoustic seals of ear canals of sizes and geometries which are not amenable to competent sealing by the premolded single-flange earplugs of the prior art. Again, however, certain problems do arise with such multiple-flange earplugs. For instance, several of the multiple-flange earplugs of commerce comprise flange elements which extend substantially perpendicularly to the stalk member to which they are affixed. Such flanges, when inserted into the usually ovoid ear canal, initially make contact with the opposed ear canal walls of the narrowest dimension and, because they extend perpendicularly from the stalk member, the insertion force required is generally substantially higher than required when a similarly sized single-flange premolded earplug is employed. Too, the edges of these perpendicularly oriented flanges tend to scrape the ear canal walls upon insertion and tend to resist removal by digging into the ear canal walls when the plug is pulled out of the ear canal. Obviously, both of these last-mentioned phenomena can be quite uncomfortable to the user.

One multiple-flange earplug which largely avoids the above problems is presently sold under the trademark "ULTRA FIT" (Cabot Safety Corporation) and falls within the scope of U.S. Patent No. 4,867,149, to Robert N. Falco, granted on September 19, 1989. This premolded, multiple-flange earplug is characterized by three spaced apart resilient solid polymeric flanges which sweep back in an arcuate hemispherical manner from the supporting stalk member and in which annular free spaces are defined between each of the flanges and the underlying supporting stalk member in order to allow the flanges to be readily deflected into said free spaces upon insertion of the plug into the ear canal. In consequence, it is the smooth arcuate exterior surfaces and not the edges of the flanges which make principal contact with the ear canal walls. Thus, insertion forces tend to be lower and the edge scraping and digging phenomena associated with perpendicularly oriented flanges are avoided. In addition, the contact area between each of the inserted hemispherical flanges and the surrounding ear canal wall is maximized. This better assures competent acoustic sealing of the ear canal for a larger overall range of ear canal sizes and geometries and with better low frequency attenuation characteristics and greater comfort than can be provided by the multiple-flange premolded earplugs having perpendicularly oriented flanges. Nevertheless, even these state-of-the-art premolded multiple-flanged earplugs exhibit some undesirable characteristics which mitigate against their universal application. Firstly, the solid resilient polymeric flanges, like those of the aforementioned single-flange plugs of the prior art, have a stable resting state shape which may not be capable of successfully accommodating the extreme flexures which may be required of them when they encounter ear canal sizes and geometries lying at the small end of the anthropometric spectrum. Generally speaking, this condition is reflected in excessive insertion forces and/or pleating of one or more of the flanges with concomitant formation of air leaks and loss of attenuation. Yet another problem may arise when such plugs are utilized with relatively small ear canals wherein, perforce, insertion occurs to a lesser extent than with medium or large ear canals. Under these circumstances a significant length of the overall earplug may extend outwardly beyond the plane of the auricle and, as a result, the user may well consider such plugs to be cosmetically obtrusive and unattractive. A still further problem can arise when a person having an early bend in the ear canal (a not uncommon anatomical occurrence) attempts to use premolded multiple-flange earplugs. In order to avoid discomfort such an individual tends to insert a multiple-flange earplug only to the point where the forwardmost flange contacts the bend in the ear canal. However, such shallow insertion often results in an inadequate seal of the canal and, consequently, inadequate attenuation. Generally, the attenuation characteristics can be substantially improved by deeper insertion of the plug into the ear canal. This can be facilitated by reaching behind the head with the opposite hand, grasping the upper rear margin of the auricle and pulling it upwardly and rearwardly while inserting the earplug. This action temporarily straightens the early bend in the ear canal to at least some significant degree and thus facilitates a reasonably deep insertion of the plug into the canal. However, when the auricle is released, the ear canal attempts to spring back to its resting shape, thereby creating a condition of impaired comfort by the resulting pressure wrought by the ear canal on the more deeply inserted earplug.

In my prior U.S. Patent Nos. 3,811,437 and Re. 29,487 there are taught certain roll-down type hearing protective earplugs composed of a slow rate of recovery polymeric foam and having a size and shape adapted to be compressed, inserted into the ear canal and therein allowed to expand to result in a comfortable and complete obturation of the ear canal. Earplugs manufactured in accordance with the aforementioned patents have met with outstanding commercial success in the marketplace due to their features of easy insertability, comfort, excellent attenuation properties and their ability to be produced in a single size while competently fitting almost the entire adult population. Nevertheless, said foam earplugs do possess certain deficiencies which mitigate against their use in certain hearing protective situations. Firstly, the earplugs of the above-identified patents are prepared for insertion by initially rolling them down between thumb and fingers, thereby to compress them to below the sizes of the ear canals into which they are to be inserted. In terms of hygiene, therefore, the user's hands should be at least relatively clean at the time of use. This is not a trivial matter because many noisy industrial environments are associated with such activities as metalworking; thus, the workers' hands are often not clean at the time of use of the plugs and the necessity for first cleansing the hands can be a bothersome requisite in such situations. Additionally, while the preliminary step of rolling the plugs between the fingers is, indeed, a simple physical step, hand disabled users, such as those suffering from arthritis, can find such step a near or actual impossibility. Moreover, the slow-recovery foam earplugs of the aforementioned patents are widely recognized as providing the highest attainable consistent attenuation of all currently available earplugs and, for that matter, often provide higher attenuation values than hearing protectors of other types. Since about 92% of the industrial noise requiring hearing protection in this country is at an intensity of 95dB or less, some consider the high degree of protection afforded by these slow recovery foam plugs as excessive.

In United Kingdom Patent No. 733,542, to Hultgren, there is disclosed a push-in type earplug comprising a soft, elastic, bullet-shaped spongy body element having a stiff axially oriented stem by which to facilitate insertion and removal of the spongy body element into and from the ear canal. Hultgren also discloses the possibility of controlling the acoustic attenuation properties of his plug by varying the pore size and density of the spongy polymeric body element. As in the case of other prior art premolded plugs, the earplugs of Hultgren are also said to be produced in two or three sizes. This deficiency is, as mentioned before, uneconomic for the manufacturer and bothersome throughout the distribution chain in terms of expense and complexity of purchasing and inventory control. In addition, in order to assure good attenuation performance, the Hultgren plugs, as in the case of any earplug which is provided in several sizes, should be fitted to the individual wearer by a skilled hearing protection specialist. Unlike my present invention, the elastic bullet-shaped spongy polymeric element of the earplug disclosed by Hultgren does not comprise a flange.

In West German OS 2 325 823, to Envac Establishment, filed on May 22, 1973 and laid open on December 19, 1974, there is disclosed an earplug comprising a spherical polymeric foam body element having an essentially impermeable outer surface and a stiff elongate handle extending therefrom. The foam body element can be comprosed of such polymeric materials a polyurethane or plasticized polyvinylchloride. As in the case of the Hultgren patent discussed above, this West German application neither discloses nor suggests the presence of a polymeric foam flange, which flange is an essential feature of the present invention.

In DE 3304362A there is disclosed a polymeric foam earplug having a cap-shaped part at one end and a spherically shaped part of smaller diameter at the other end the parts being releasably connected by a pin element carried on the spherical part engaging in a short hollow portion of the cap-shaped part.

It is a principal object of the invention to provide a new and novel single-flange hearing protective earplug construction.

It is another object of the invention to provide a single-flange push-in type earplug construction which may be produced in a single size, but which provides easy insertion, wearer comfort and good sound attenuation to substantially the entire adult population.

It is another object of the invention to provide a single-flange push-in type hearing protective earplug construction having the desirable performance characteristic of relatively linear attenuation as a function of frequency over the audible range of frequencies, thereby to allow the wearer thereof to perceive sound without the hollow muffled quality usually perceived by wearers of earplugs having non-linear (relative to frequency) attenuation characteristics.

It is still another object of the invention to provide a new and novel earplug construction which may be utilized either as a push-in or roll-down plug.

Other objects and advantages of the invention will in part be obvious and will in part appear hereinafter.

The earplug of the invention comprises a resilient polymeric foam body element having the features of the precharacterising portion of claim 1. In the earplug of the present invention the elongate stem is of a material different from the foam body element and has a stiffness greater than that of the foam body element and sufficient to allow manipulation of the foam body element into a wearer's ear canal, one end of which stem is secured to the interior of the nose of the body element to avoid separate of the stem from the body element during use, the said earplug having an Insertion Force value of between 40 and 350g, wherein the said earplug can be utilized in alternative insertion modes, either as a push-in or a roll-down type earplug.

### BRIEF DESCRIPTION OF THE DRAWING

Figure 1 hereof is a side view, partly in section, of an earplug in accordance with the invention.

Figure 2 is a side view, partly in section, of another embodiment of an earplug in accordance with the invention.

Figures 3A through 3D, inclusive, are perspective views, with Figure 3C partly in section, illustrating one method by which a thermoformable resilient soft polymeric foam sheet material may be fabricated into earplugs in accordance with the invention.

Figure 4 is an ANSI S 12.6 plot attenuation performance relative to frequency of an embodiment of the invention prepared in accordance with the working example hereof.

Figure 5 is a top view of a gauge suitable for determining the Insertion Force value of an earplug in accordance with the invention.

Figure 6 is a sectional view of the gauge of Figure 5, taken through lines VI-VI thereof.

Figure 7 is a sectional view of the gauge of Figure 5, taken through line VII-VII thereof.

### DESCRIPTION OF THE PREFERRED EMBODIMENTS

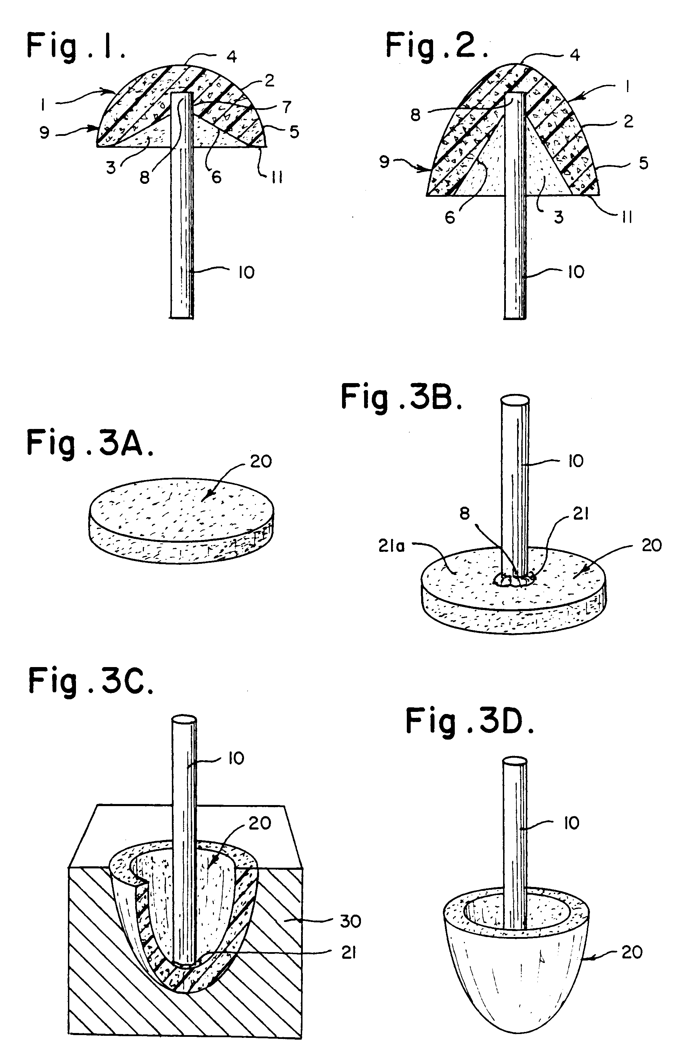

Referring now particularly to Figures 1 and 2 hereof, wherein like reference numerals refer to like structures, the earplug construction of the invention broadly comprises a soft resilient polymeric foam body element 1 and an elongate stem 10 extending rearwardly and axially therefrom. While the cross sectional shape of the particular embodiments shown in Figures 1 and 2 is circular, it will be appreciated that said cross sectional shape may also be ovoid or elliptical. In any event, in order to achieve the essentially universal fit benefit of the invention and depending somewhat upon the density and softness of the polymeric foam of which it is composed and the specific geometry of the foam body element 1, the maximum cross sectional exterior dimension of the body element 1 at any point along its length, whether of circular or ovoid cross section, can be up to about 13.72 mm (0.540 inch).

The body element 1 comprises a rounded nose or forward end 4 and, coextensive therewith, a skirt 5 extending rearwardly therefrom and terminating in a trailing edge 11. The skirt 5 defines the flange element 9 of the plug construction and a hollowed out base cavity 3. The interior surface 6 of skirt 5 may, but need not, conform precisely to its outer surface 2. The length of the body element 1 relative to its maximum diameter is subject to considerable variation and is not normally critical. For example, the external shape of body element 1 of the earplug shown in Figure 1 is substantially hemispherical, therefore, its length to diameter ratio is about 0.5 to 1. Contrastingly, in the embodiment of Figure 2 the body element 1 is shown to be distinctly bullet shaped and has a length to diameter ratio of about 3 to 2.

Centrally located on the interior of nose end 4 is a recess 7 to receive the free end 8 of an elongate stem 10 which extends axially and rearwardly to a point outside the trailing edge 11 of skirt 5. It is usually desirable that the thickness of the polymeric foam material at the nose end 4 of body element 1 be at least somewhat thicker than that of the trailing edge 11 of skirt 5, thereby to provide a recess 7 of sufficient depth as to securely capture the end 8 of stem 10 without compromising the structural integrity of the body element 1 at the nose end 4 thereof. For this reason, coupled with consideration of easy mold design and forming operations, it is generally preferred that the shape of the cavity 3 of the body element 1 be conical, the base thereof being coextensive with the trailing edge 11 of skirt 5.

There exists a complex and as yet incompletely understood interplay between the density, softness and resilience of the foam material of construction of the body element 1, its diameter and size, and the size, depth and geometry of the base cavity 3. Generally speaking, the softer the foam material of construction, the lower its density and/or the lower its resilience, the shallower can be the base cavity 3 and the shorter and/or thicker can be the skirt 5. Conversely, the stiffer the foam material of construction, and/or the greater the density thereof and/or the greater its resilience, the greater need be the depth and volume of the base cavity 3 and the longer and thinner need be the skirt 5 required to attain the benefits of the invention. The Insertion Force test procedure described below takes these factors into account without a detailed inquiry into the contribution of each to the functional properties of the overall earplug construction. The practitioner of the invention can employ the test to good advantage in the design and development of earplugs in accordance with the invention and can readily determine, with all necessary precision, whether candidate plugs fall within the ambit of the invention. Utilizing the Insertion Force test, one of ordinary skill can provide a few prototypal samples of a candidate earplug, determine the Insertion Force value thereof and, if the prototypal plug does not fall within the Insertion Force value range of the invention, readily alter one or more of the foregoing parameters appropriately in order to achieve the invention.

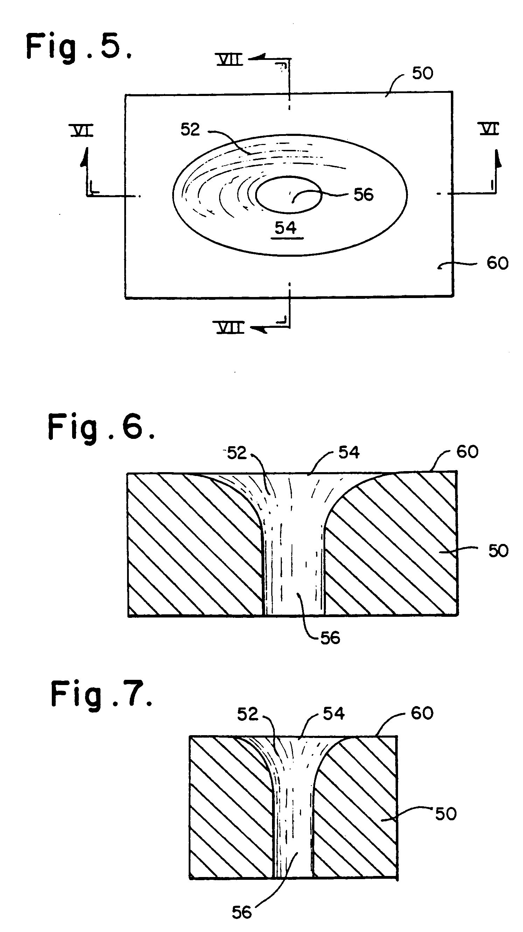

The Insertion Force test referred to above involves a gauge having a well defined elliptical aperture machined thereinto which approximates the size and shape, but not the tortuosity, of an average human ear canal. Referring now to Figures 5, 6 and 7, the gauge comprises a gauge block 50 composed of polymethylmethacrylate (or similar hard plastic material) and having an elliptical aperture 52. The final gauge bore 56, which starts at a depth of 5.08 mm (0.200 inch) from the surface 60 of the gauge block 50, has a major axis of 11.05 mm (0.435 inch) and a minor axis of 7.42 mm (0.292 inch). The mouth 54 of elliptical aperture 52 has a major axis of 24.58 mm (0.968 inch), a minor axis of 21.00 mm (0.827 inch) and is gently radiused about its circumference to transition smoothly into the final gauge bore 56. The length of the final gauge bore 56 is at least sufficient to enable the entry of the entire body element 1 thereinto during the test procedure. In general, I have found that a bore 56 length of about 11.78 mm (0.464 inch) is adequate for most candidate earplugs.

In carrying out the Insertion Force test, the foam body element 1 of the candidate earplug is first lightly talced and the nose end of the talced plug rested against the mouth 54 of the gauge block 50 with the stem 10 thereof vertically and centrally located over the final gauge bore 56. Then, an axial downward force is applied to the stem 10 sufficient to slowly drive the body element 1 thereof completely into the final gauge bore 56. This force is continuously measured throughout the insertion step by any suitable means and the maximum or peak force experienced during insertion, expressed in grams, is taken as the Insertion Force value. A simple method for measuring the applied force comprises placing the gauge block 50 and the candidate earplug on the balance pan of an electronic taring or "zeroing" balance, zeroing the resulting weights thereof and then manually inserting the candidate earplug into the gauge while continuously observing and/or recording the sensed balance pan weight until the body element 1 of the candidate earplug is driven completely into the final gauge bore 56. Of course, if desired, substantially more esoteric test apparatus, such as an Instron tensile test apparatus equipped with a suitable load cell, may be suitably employed in carrying out the force measurement of the Insertion Force test procedure.

Utilizing the test procedure and gauge block described above, singe-flange earplugs of the invention will have an Insertion Force value of between about 40 and about 350 grams. Where the Insertion Force value is greater than about 350 grams, user comfort during insertion and/or wearing of the plug is compromised. Where the Insertion Force value is less than about 40 grams, the attenuation performance of the plug, particularly at low frequencies, can be found inadequate for many environmental noise situations encountered. In a preferred embodiment of the invention, the Insertion Force value of the earplug will fall within the range of 60 grams and 160 grams.

The relative hardness value of a small ware composed of a soft resilient polymeric foam is generally difficult to measure with precision by the Shore 00 Durometer technique. However, with the sole intention of providing a general guideline and with no intention whatsoever of limiting the invention, I have so far found that instantaneous Shore 00 Durometer values of acceptable foams for use as the body element 1 in the present invention appear to be no greater than about 60 Durometer units and have usually been found to reside within the range of from about 20 to about 45 Durometer units. By "instantaneous" value, it is meant that the penetration of the Durometer indentor foot is taken immediately after application of the foot load to the sample and without affording the foam sample time to substantially creep or relax under the load of the indentor foot subsequent to its application.

The relative stiffness of a polymeric foam material is generally dependent upon the basic molecular structure thereof (such as crosslink density, branching, crystallization, the length and density of hard segments versus soft segments in the polymer chain, i.e., internal plasticization, etc.), the types and quantities of additives present in the foam formulation (such as, external plasticizers, fillers and reinforcing agents) and the apparent density of the foam material. In general, I prefer that the apparent density of the body element 1, as calculated from suitable volume and weight measurements thereof, be no greater than about 0.32 g/cm³ (20 lbs/ft³) and, even more preferably, no greater than about 0.24 g/cm³ (15 lbs/ft³).

The resilience of the polymeric foam material of construction of the body element 1 is sufficient as to ensure substantially complete recovery of the original shape and size of said body element when temporarily deformed and released in free space. While polymeric foam materials which are rubbery, and thus recover fully and substantially instantaneously from deformation, may be employed to good effect in the present invention, I prefer that the foam employed for the construction of the body element 1 have retarded recovery characteristics, that is to say, that it recovers completely, but relatively slowly, when a deforming stress is removed therefrom. Such foams are said to exhibit viscoelastic, as opposed to elastic, behavior. When such viscoelastic foams are employed in the construction of the body element 1 there results an earplug construction which can be utilized either as a push-in or a roll-down type earplug. In the push-in mode, the body element 1 composed of a viscoelastic polymeric foam can simply be forced into the ear canal by suitable manipulation of the stem 10. However, should the user desire it or should the ear canals of the user be exceptionally small or tortuous, thereby rendering the push-in mode of operation difficult or undesirable, the user can then use an alternate method of insertion in a manner akin to that described in my U. S. Pat. Re. No. 29,487. Thus the viscoelastic foam body element 1 can be rolled axially between thumb and fingers so as to compress same to below the cross section of the ear canal into which it is to be inserted. Then, arising from the viscoelastic or slow recovery behavior thereof, the user is afforded sufficient time to insert the body element 1 into the ear canal by suitable manipulation of the stem 10 before recovery of the body element 1 occurs to the extent of interference thereof with the enclosing ear canal wall.

A simple and adequate test for 90% recovery time for purposes of the present invention is to provide a glass or clear plastic tube having an internal diameter 90% of the maximum cross sectional dimension of the body element 1 under consideration. The body element 1 of the earplug is then rolled down tightly and lengthwise between thumb and fingers for about one minute and immediately thereafter inserted into the tube. At the instant of release of the body element into the tube a stopwatch is started, the tube turned upright over a suitable support surface (such as a table top) and the tube cycled vertically close to and over the support surface at a cyclic rate of approximately 2/second and at an amplitude of approximately 1/4 inch (6.35 mm). The cycling is undertaken such that the free end of the stem 10 of the plug remains in continuous contact with the support surface until such time as the recovering body element makes sufficient contact with the enclosing tube as to ride along with said tube during its vertical motion as opposed to sliding freely therewithin. The time for this to occur is noted and is taken as the 90% recovery time. The test procedure is carried out on at least three and preferably at least five sample earplugs of the candidate design and foam formulation in order to establish statistical significance and the results then averaged to provide an average 90% recovery time for the lot under test. Using this procedure, the preferred viscoelastic polymeric foam body element 1 of the present invention will have an average 90% recovery time of between about 2 and about 120 seconds.

There are many resilient polymeric foam compositions capable of meeting the requirements of the present invention, including the desirable, viscoelastic or slow recovery properties mentioned above. For instance, many of the externally and internally plasticized polymer foams disclosed in my U. S. Patent Re. No. 29,487 are generally suitable for use as a material of construction of the body element 1. So, too, are many of the polyurethane foam compositions disclosed in U. S. Patent No. 4,158,087, to Louis Leonard Wood, June 12, 1979, entitled, "Urethane Foams Having Low Resiliency".

Polyurethane foam compositions are generally preferred for the body element 1 due to their formulation flexibility, easy molding characteristics and economics. Of these, polyether polyurethane foams are even further preferred due to the generally soft surface "hand" or feel of resilient foam wares produced therewith. The polyether polyurethane foam compositions based on polyurethane prepolymers blended with acrylic latex modifiers in accordance with the above-mentioned Wood Patent have been found to be useful in the invention. Such polyether polyurethane prepolymers are currently available from W. R. Grace Company under the "HYPOL" brand name. Suitable acrylic latex modifiers are available from Union Carbide Corporation under the "UCAR" brand name.

Stem 10 is an elongate member which may be tubular or solid throughout its cross section and may be uniform or slightly tapered along its length. In the case of a slightly tapered configuration, the larger end will normally be utilized as the end 8 inserted into recess 7 of the foam body element 1. In any event, the cross sectional dimension of stem 10 at said end 8 should not be so great as to prevent easy insertion of the earplug into ear canals of small size or to interfere with the deflection of the skirt 5 into the concavity 3 as the body element 1 is pushed into the user's ear canal. To this end, the maximum cross sectional dimension of the stem 10 at end 8 should be within the range of from 1.6 mm (0.0625 inch) to 4.8 mm (0.1875 inch) and I prefer that said dimension be within the range of from 3.2 mm (0.125 inch) to 4.0 mm (0.156 inch). Where the maximum cross sectional dimension of the stem 10 at end 8 resides within the lower portion of the foregoing range, insertion of the body 1 into small size ear canals is facilitated. On the other hand, where the maximum cross sectional dimension of the stem 10 at end 8 resides within the upper portion of the foregoing range, the resulting earplug generally produces less pressure on the ear canal during insertion and tends to improve the sense of comfort felt by the wearer. The stem 10, of course, can be of any convenient length such as to provide a conveniently manipulable insertion and removal member for the foam body element 1; thus, the exact length selected for the stem 10 will largely be a matter of choice. Of course, the dimensions, geometry and material of construction employed for the stem 10 are selected such that the stem 10 will be sufficiently stiff as to allow manipulation of the foam body element 1 into the ear canal.

Many stem 10 constructions will suggest themselves to those of skill in the art and are suitable. For instance, paper, rubber, cardboard and plastic rod forms of the type often used as medical cotton swab holders are generally of adequately stiff nature and appropriate cross sectional dimension for use in the present earplug construction. So, too, are many known plastic drinking and stirring straws. Whatever the selection of the stem 10 material, however, it is important that the end 8 thereof be attached to the foam body element 1 with sufficient security as to avoid separation of the stem 10 from the body 1 during use. The security of attachment can be achieved in any suitable manner, such as by use of suitable adhesives or by solvent or thermal welding. In a preferred embodiment of the invention, the foam body element 1 is composed of a molded polyurethane foam and the stem 10 is affixed to the body element 1 during the molding operation. For example, the stem 10 can be utilized as an insert in the mold for the foam body element 1, the end 8 thereof acting as a male mold member for recess 7 and, as the foam formulation of the foam body element 1 blows and cures in the mold it bonds firmly and tenaciously to the end 8 of the stem 10. Where this method is employed, I further prefer that the stem 10 be composed of bonded paper since it has been found to tenaciously bond to curing polyurethane polymer compositions.

While a preferred method of manufacture, the body element 1 of the earplug of the invention need not be prepared by in-mold foaming of a precursor polymer composition. A suitable alternative manufacturing scheme is depicted in Figures 3A through 3D hereof, to which reference is now made. Here, the starting material is a thermoformable polymeric foam in sheet form, such as a plasticized polyvinylchloride foam material. As shown in Figure 3A, there is first provided a disk 20 of the thermoformable sheet foam of appropriate dimensions. Next, as shown in Figure 3B, the end 8 of a suitably dimensioned stem 10 is affixed to the geometric center of the upper surface 20a of said disk 20, such as by means of a suitable cement weldment 21. The stem 10 is, of course, oriented such as to be perpendicular to the surface 20a. In Figure 3C there is shown the disk 20 forced into a mold 20 by application of axial insertion force to the affixed stem 10. The thermoformable polymeric foam material of the disk 20 is heated to thermoforming temperatures, either by preheating thereof preparatory to insertion into the mold 30 or by suitable heating of the mold 30. The heating is accomplished to the extent that the thermoformable polymeric foam material of the disk 20 can relax or creep, but not to the extent that fluxing or collapse of the cellular structure thereof occurs. Of course, some tolerable amount of densification of the foam material can take place during the thermoforming step. Next, the material of disk 20 is cooled to below thermoforming temperature while in the mold 30 and the completed earplug is then removed therefrom, as shown in Figure 3D.

There follows an illustrative, non-limiting example.

### EXAMPLE

Earplugs in accordance with Figure 1 hereof are produced by molding the foam body elements 1 thereof in a multi-cavity aluminum mold using a polyetherpolyurethane foam precursor composition containing a self-crosslinking, acrylic latex modifier. The mold comprises a closure plate having an aperture overlying the center of each body element 1 cavity and through which aperture there is received a stem element 10. Upon injection of the polyetherpolyurethane precursor charges into the respective mold cavities, the cover is placed over the mold, thereby bringing the end 8 of each stem element captured in the mold cover apertures to a distance of 2.16 mm (0.085 inch) from the nose end of the mold cavity. The polyetherpolyurethane precursor charge in each cavity blows and partially cures in the closed cavity, the curing and blowing charge thereby filling the cavity, forming the body element 1 and recess 7 and, at the same time, bonding the end 8 of the stem 10 to the polyurethane material of the recess 7. The filling and curing of the mold cavities is undertaken at ambient temperature and, after 6-8 minutes in the closed mold, the closure plate is removed and the finished earplug wares removed, placed in a fabric bag and dried for 40 minutes in a clothes dryer set at the "Medium" setting in order to remove excess water from the formed wares and to complete the curing of the body elements 1. The particular polyether polyurethane foam precursor formulation employed was as follows:

| Ingredient | Parts by Weight |
|---|---|
| HYPOL 2002, polyether polyurethane prepolymer | 100 |
| UCAR 154, self cross-linking acrylic resin latex | 79.2 |
| Water with 8.8 weight percent Pluronic F68 surfactant (BASF Wyandotte) | 20 |
| Sun Yellow YFD 1123 colorant (Sun Chemical Company) | 1 |

The stem 10 elements were composed of a rolled bonded paper rod stock having a uniform diameter of 4.0 mm (0.156 inch) and a length of 25.7 mm (1.013 inch).

The resulting cured earplugs in accordance with Figure 1 hereof had the following dimensions:

| | |
|---|---|
| Length of body element 1 | 6.6 mm (0.260 inch) |
| Maximum diameter of body element 1, taken at trailing edge 11 of skirt 5 | 13.2 mm (0.520 inch) |
| Exterior shape of body element 1 | hemispherical, circular cross section |
| Thickness of skirt 5 at trailing edge 11 | 2.3 mm (0.091 inch) |
| Depth of recess 7 | 2.2 mm (0.088 inch) |
| Shape of base cavity 3 | conical |
| Diameter of base cavity 3 taken at trailing edge 11 | 8.6 mm (0.338 inch) |
| Diameter of base cavity 3 taken at shoulder of recess 7 | 4.0 mm (0.156 inch) |
| Minimum vertical distance from shoulder of recess 7 to trailing edge 11 | 2.5 mm (0.100 inch) |

The instantaneous Shore 00 Durometer value was determined by separating the foam body element 1 from two of the earplugs and stacking the two separated body elements 1 under the indentor foot of the Durometer in such manner that the foam material of the one body element 1 lay beneath and in contact with the foam material of the second body element 1. Then, the Durometer value was taken substantially immediately after application of the indentor foot load to the contacting materials and was found to be about 35 Durometer 00 units.

The density of the foam body element 1 was determined by direct dimensional and weight measurements thereof. The foam body element 1 was first conditioned at room temperature and 50% relative humidity for at least about 24 hours. It was then weighed on an analytical balance capable of resolution of 0.0001 gm. Dimensional measurements were taken using an optical comparator or microscope at a magnification of at least 10 X. The volume of the body element 1 was calculated from said measurements. In the particular embodiment hereof, referring to Figure 1, the volume was calculated by first determining the overall volume of the hemispherical body and subtracting from this volume the calculated volumes of the conical base cavity 3 and the cylindrical recess 7. Using this protocol, the density of the foam body element 1 was found to be 0.168 gm/cm³ (10.5 lbs/ft³).

The average 90% recovery time of the earplugs was determined as disclosed hereinbefore, utilizing as a gauge a cylindrical polymethylmethacrylate tube having an internal diameter of 11.8 mm (0.468 inch). The average 90% recovery time was determined to be 12.3 seconds.

Samples of the earplugs were subjected to the Insertion Force test previously disclosed and the Insertion Force value thereof was found to be about 116 grams.

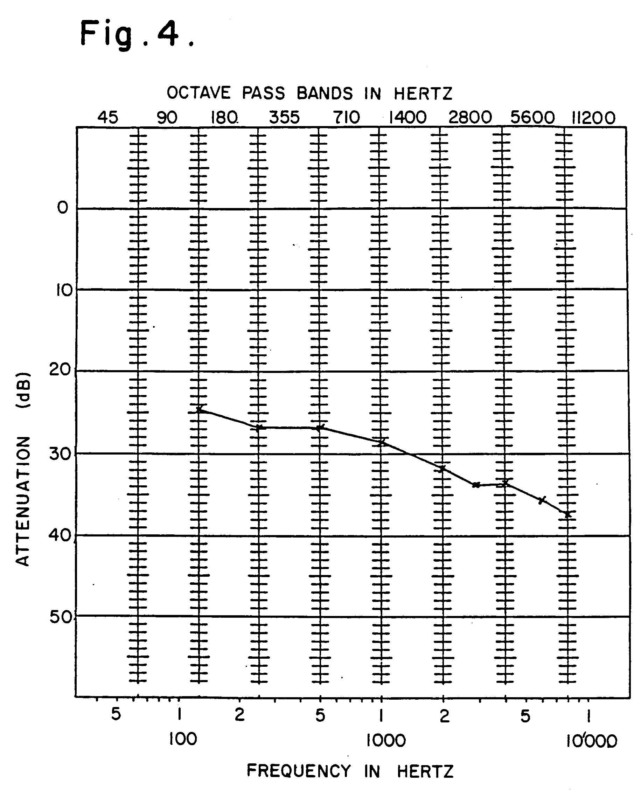

The earplugs of this example were subjected to attenuation tests undertaken under the protocol of ANSI S12.6 (American National Standards Institute). The results of said tests are depicted in the plot of Figure 4 hereof, which discloses the relatively linear attenuation provided by the plug of the invention as a function of frequency.

Several human subjects, having widely varying ear canal sizes and geometries, utilized the earplugs of this example in noisy industrial environments. All subjects reported that the earplugs were easy to use, whether by the push-in or roll-down modes of insertion, were comfortable throughout insertion, wearing and removal and provided adequate attenuation for their needs. The subjects also reported that the sounds perceived by them during their wearing of the earplugs of this working example were of substantially more normal quality than experienced when the subjects wore properly fitted earplugs of the three-flange type.

## Claims

1. A hearing protective earplug comprising: a resilient polymeric foam body element (1) of circular or ovoid cross section having a maximum diameter of no greater than 13.73 mm, said body element (1) comprising a rounded nose end (4) and, coextensive therewith, a rearwardly directed skirt (5) defining a flange (9) having a trailing edge (11), the interior surface (6) of said skirt (5) defining a base cavity (3), said base cavity (3) being of a size and shape sufficient to allow said skirt (5) to deflect inwardly under the urging of an ear canal in to which it is inserted and to establish an acoustic seal therewith without puckering of the exterior surface (2) of said body element (1), said earplug includes an elongate stem (10) extending axially and rearwardly from said nose end (4) to a point outside the trailing edge (11) of said skirt (5), characterised in that the elongate stem is of a material different from the foam body element (1) and has a stiffness greater than that of the foam body element (1) sufficient to allow manipulation of the foam body element into a wearer's ear canal, one end (8) of which stem is secured to the interior of the nose end (4) of said body element (1) to avoid separation of said stem (10) from the body element during use, the said earplug having an Insertion Force value of between 40 and 350g, wherein said earplug can be utilized in alternative insertion modes, either as a push-in or a roll-down type earplug.

2. The earplug of claim 1 wherein said polymeric foam body element (1) is composed of a viscoelastic polymeric foam.

3. The earplug of claim 2 wherein said polymeric foam body (1) element has a 90% recovery time of between about 2 and about 120 seconds.

4. The earplug of claim 1 wherein the apparent density of the polymeric foam body element (1) is no greater than about 0.32 g/cm³.

5. The earplug of claim 1 wherein the apparent density of the polymeric foam body element (1) is no greater than about 0.24 g/cm³.

6. The earplug of claim 1 wherein said polymeric foam body element (1) has an exterior shape (2) which is hemispherical and the length to diameter ratio thereof is about 0.5:1.

7. The earplug of claim 1 wherein the exterior shape (2) of said polymeric foam body element (1) is bullet shaped.

8. The earplug of claim 1 wherein the shape of said cavity (3) is conical, the base thereof being coextensive with said trailing edge (11).

9. The earplug of claim 1 wherein said polymeric foam body element (1) is composed of a polyurethane.

10. The earplug of claim 9 wherein said polyurethane is a polyether polyurethane.

11. The earplug of claim 9 wherein said polyether polyurethane foam is a viscoelastic foam containing an acrylic latex modifier.

12. The earplug of claim 1 wherein said Insertion Force value of said earplug is between about 60 and 160 grams.

13. The earplug of claim 9 wherein said stem element (10) is composed of a bonded paper material.

14. The earplug of claim 1 wherein the maximum cross sectional dimension of said stem element (10) at the point of attachment thereof with said polymeric foam body element (1) is between about 3.2mm and about 4.0 mm.

15. The earplug of claim 9 wherein said polyurethane foam body element (1) is formed by molding of a polyurethane foam precursor formulation and wherein said stem element (10) is secured thereto during said molding.

16. The earplug of claim 15 wherein said polyurethane foam precursor formulation contains an acrylic latex modifier.

## Patentansprüche

1. Ohrstöpsel als Gehörschutz, der aufweist: ein elastisches, polymeres Schaumstoffkörperelement (1) mit kreisförmigem oder ovalem Querschnitt, das einen maximalen Durchmesser nicht größer als 13,73 mm besitzt, wobei das Körperelement (1) ein abgerundetes Nasenende (4) und mit gleichem Umfang dazu einen nach hinten gerichteten Rand (5) aufweist, der einen Flansch (9) definiert, der eine Hinterkante (11) besitzt, wobei die Innenoberfläche (6) des Rands (5) einen. Basishohlraum (3) definiert, wobei der Basishohlraum (3) von einer Größe und Form ist, die ausreichend ist, um dem Rand (5) zu ermöglichen, daß er sich nach innen unter dem Drücken eines Ohrkanals, in den er eingesetzt wird, ablenkt und um eine akustische Dichtung damit ohne ein Kräuseln der Außenoberfläche (2) des Körperelements (1) vorzunehmen, wobei der Ohrstöpsel einen langgestreckten Schaft (10), der sich axial nach hinten von dem Nasenende (4) zu einem Punkt außenseitig der Hinterkante (11) des Rands (5) erstreckt, umfaßt, gekennzeichnet dadurch, daß der langgestreckte Schaft aus einem Material unterschiedlich zu demjenigen des Schaumstoffkörperelements (1) ist und eine Steifigkeit größer als diejenige des Schaumstoffkörperelements (1) besitzt, die ausreichend ist, um eine Manipulation des Schaumstoffkörperelements in einen Ohrkanal eines Tragenden hinein zu ermöglichen, wobei ein Ende (8) des Schafts an dem Inneren des Nasenendes (4) des Korperelements (1) gesichert ist, um eine Trennung des Schafts (10) von dem Körperelement während der Benutzung zu vermeiden, wobei der Ohrstöpsel einen Einsetzkraft-Wert zwischen 40 und 350 g besitzt, wobei der Ohrstöpsel, in alternativen Einsetz-Moden, entweder als ein Ohrstöpsel vom Eindrück- oder vom Zusammenroll-Typ, verwendet werden kann.

2. Ohrstöpsel nach Anspruch 1, wobei das polymere Schaumstoffkörperelement (1) aus einem viskoelastischen, polymeren Schaumstoff zusammengesetzt ist.

3. Ohrstöpsel nach Anspruch 2, wobei das polymere Schaumstoffkörperelement (1) eine 90% Erholungszeit zwischen ungefähr 2 und ungefähr 120 Sekunden besitzt.

4. Ohrstöpsel nach Anspruch 1, wobei die auftretende Dichte des polymeren Schaumstoffkörperelements (1) nicht größer als ungefähr 0,32 g/cm³ beträgt.

5. Ohrstöpsel nach Anspruch 1, wobei die auftretende Dichte des polymeren Schaumstoffkörperelements (1) nicht größer als ungefähr 0,24 g/cm³ beträgt.

6. Ohrstöpsel nach Anspruch 1, wobei das polymere Schaumstoffkörperelement (1) eine Außenform (2) besitzt, die halbkugelförmig ist, und das Verhältnis von Länge zu Durchmesser davon ungefähr 0,5:1 beträgt.

7. Ohrstöpsel nach Anspruch 1, wobei die Außenform (2) des polymeren Schaumfstoffkörperelements (1) geschoßförmig ist.

8. Ohrstöpsel nach Anspruch 1, wobei die Form des Hohlraums (3) konisch ist, wobei die Basis davon von gleichem Umfang wie die Hinterkante (11) ist.

9. Ohrstöpsel nach Anspruch 1, wobei das polymere Schaumstoffkörperelement (1) aus einem Polyurethan besteht.

10. Ohrstöpsel nach Anspruch 9, wobei das Polyurethan ein Polyetherpolyurethan ist.

11. Ohrstöpsel nach Anspruch 9, wobei der Polyetherpolyurethan-Schaumstoff ein viskoelastischer Schaumstoff ist, der einen acrylischen Latex-Modifizierer enthält.

12. Ohrstöpsel nach Anspruch 1, wobei der Einsetzkraft-Wert des Ohrstöpsels zwischen ungefähr 60 und 160 Gramm liegt.

13. Ohrstöpsel nach Anspruch 9, wobei das Schaftelement (10) aus einem verbundenen bzw. verklebten Papiermaterial besteht.

14. Ohrstöpsel nach Anspruch 1, wobei die maximale Querschnittsdimension des Schaftelements (10) an dem Punkt der Befestigung davon mit dem polymeren Schaumstoffkörperelement (1) zwischen ungefähr 3,2 mm und ungefähr 4,0 mm liegt.

15. Ohrstöpsel nach Anspruch 9, wobei das Polyurethanschaumstoffkörperelement (1) durch Gießen einer Polyurethan-Schaumstoff-Präkursor-Zubereitung gebildet wird und wobei das Schaftelement (10) daran während des Gießens befestigt wird.

16. Ohrstöpsel nach Anspruch 15, wobei die Polyurethan-Schaumstoff-Präkursor-Zubereitung einen acrylischen Latex-Modifizierer enthält.

## Revendications

1. Protège-tympan comprenant un élément de corps (1) en une mousse de polymère élastique, à section transversale circulaire ou ovale ayant un diamètre maximum ne dépassant pas 13,73 mm, ledit élément de corps (1) comprenant une extrémité de nez (4) arrondie et venue d'une pièce avec une jupe (5) s'étendant suivant la tigre et orientée vers l'arrière pour former une bride (9) ayant un bord arrière (11), la surface interne (6) de ladite jupe (5) délimitant une cavité de base (3), ladite cavité de base (3) ayant une taille et une forme adaptées pour permettre à ladite jupe (5) de se déformer vers l'intérieur, par l'effet de la paroi du canal auditif, au moment de l'insertion, et de former un joint étanche avec la paroi du point de vue acoustique, sans formation de fronces sur la surface externe (2) dudit élément de corps (1), et ledit protège-tympan comportant une tige allongée (10) s'étendant axialement et vers l'arrière depuis ladite extrémité de nez (4) jusqu'à un point à l'extérieur du bord arrière (11) de ladite jupe (5), caractérisé en ce que la tige allongée est en un matériau différent de celui de l'élément de corps (1) en mousse, qu'elle a une rigidité supérieure à celle de l'élément de corps (1) en mousse et suffisante pour permettre la manipulation de l'élément de corps en mousse dans le canal auditif de l'utilisateur, une extrémité (8) de ladite tige étant fixée à l'intérieur de l'extrémité de nez (4) dudit élément de corps (1) pour éviter une séparation de ladite tige (10) de l'élément de corps pendant l'utilisation, et ledit protège-tympan présentant une valeur de la force d'insertion allant de 40 à 350 g, et que ledit protège-tympan peut être inséré de différentes manières : on peut simplement le pousser ou le pousser en le tournant.

2. Protège-tympan selon la revendication 1, où ledit élément de corps (1) en mousse de polymère est constitué d'une mousse de polymère viscoélastique.

3. Protège-tympan selon la revendication 2, où ledit élément de corps (1) en mousse de polymère a un temps de récupération à 90% entre environ 2 et environ 120 secondes.

4. Protège-tympan selon la revendication 1, où la densité apparente de l'élément de corps (1) en mousse de polymère ne dépasse pas environ 0,32 g/cm³.

5. Protège-tympan selon la revendication 1, où la densité apparente de l'élément de corps (1) en mousse de polymère ne dépasse pas environ 0,24 g/cm³.

6. Protège-tympan selon la revendication 1, où ledit élément de corps (1) en mousse de polymère a une forme externe (2) qui est hémisphérique et qui présente un rapport longueur sur diamètre d'environ 0,5:1.

7. Protège-tympan selon la revendication 1, où la forme externe (2) dudit élément de corps (1) en mousse de polymère est celle d'une balle de fusil.

8. Protège-tympan selon la revendication 1, où la forme de ladite cavité (3) est conique, la base du cône coïncidant avec ledit bord arrière (11).

9. Protège-tympan selon la revendication 1, où ledit élément de corps (1) en mousse de polymère est constitué de polyuréthane.

10. Protège-tympan selon la revendication 9, où ledit polyuréthane est un polyuréthane basé sur un polyéther.

11. Protège-tympan selon la revendication 9, où ladite mousse de polyuréthane basé sur un polyéther est une mousse viscoélastique contenant un latex acrylique comme modificateur.

12. Protège-tympan selon la revendication 1, où ladite valeur de la force d'insertion dudit protège-tympan est entre 60 et 160 grammes.

13. Protège-tympan selon la revendication 9, où ledit élément de tige (10) est constitué d'un matériau à base de papier collé.

14. Protège-tympan selon la revendication 1, où ladite dimension transversale maximale dudit élément de tue (10) à son point de fixation sur ledit élément de corps (1) en mousse de polymère se situe entre 3,2 mm environ et 4,0 mm environ.

15. Protège-tympan selon la revendication 9, où ledit élément de corps (1) en mousse de polyuréthane est formé en moulant une préparation de précurseurs de mousse polyuréthane et où ledit élément de tige (10) est fixé au corps durant ledit moulage.

16. Protège-tympan selon la revendication 15, où ladite formulation de précurseurs de mousse de polyuréthane contient un latex acrylique comme modificateur.
